# EUROPÄISCHE PATENTSCHRIFT

(11) **EP 1 169 981 B1**
(45) Veröffentlichungstag und Bekanntmachung des Hinweises auf die Patenterteilung: **15.09.2004**
(21) Anmeldenummer: 01114091.0
(22) Anmeldetag: 09.06.2001
(51) Int. Cl.: A61F 2/46

(54) **Kugelabziehvorrichtung für Schaftprothesen**
Ball head pulling device for a shaft prosthesis
Dispositif pour extraire une tête sphérique d'une prothèse de tige

(30) Priorität: 07.07.2000 EP 00810596
(43) Veröffentlichungstag der Anmeldung: 09.01.2002
(73) Patentinhaber: Centerpulse Orthopedics Ltd., 6340 Baar (CH)
(72) Erfinder: Willi, Thomas, 8309 Breite Nürensdorf (CH); Baum, Ines, 78467 Konstanz (DE)
(74) Vertreter: Manitz, Finsterwald & Partner Gbr

(56) Entgegenhaltungen:
- WO-A-93/02642
- DE-C- 4 441 870
- US-A- 3 801 989

## Beschreibung

Die Erfindung handelt von einer Kugelabziehvorrichtung für Schaftprothesen mit auf den Prothesenhals aufsetzbaren Kugeln, wie z.B. aus der Patentschrift DE 44 41 870 C bekannt.

Bei Reoperationen von Hüftgelenken kommt es vor, dass ein Femurschaft noch so gut verankert ist, dass ein neuer Schaft nicht notwendig ist. In einem solchen Fall genügt es, die Gelenkkugel vom Prothesenschaft zu entfernen und eine neue Gelenkkugel aufzusetzen, die beispielsweise mit einem vollständig neuen Acetabulum kombiniert wird.

Bis anhin wurden Gelenkkugeln, die mit einem konischen Presssitz auf einem Femurschaft aufgesetzt sind, mit einem Hilfswerkzeug umfasst und durch vorsichtiges Ausschlagen mit einem Gleithammer entfernt. Dieses Ausschlagen an einem intakten und im Knochen verankerten Schaft stellt ein unnötiges Risiko für eine Lockerung des Schaftes dar, da die Haftung der Kugel auf dem Schaft nicht genau vorhersehbar ist.

Aufgabe der Erfindung ist es, einfache Instrumente zu schaffen, welche eine schonende Entfernung der Kugel sicherstellen. Dies wird dadurch erreicht, dass die Vorrichtung zwei in einem Gehäuse drehbar abgestützte Drehkörper aufweist, die jeweils mit einem Hebelarm drehbar sind und die mit ihren Drehachsen in einer gemeinsamen Ebene F liegen und in der Projektion auf die Ebene F einen geöffneten Spalt S für das Einführen des Prothesenhalses bilden, wobei die Drehkörper ein Profil aufweisen, welches mit zunehmender Drehung den Spalt S zum Klemmen des Prothesenhalses verringert und eine Vergrösserung des Abstandes ihrer Oberkante zur Ebene F bewirkt, um direkt oder indirekt über einen Zwischenkörper an der aufgesetzten Kugel eine Abziehkraft P zu erzeugen.

Der Vorteil dieser Vorrichtung liegt darin, dass sie für verschiedenste Ausführungen von Prothesenschäften verwendbar ist, da am Prothesenhals eine Klemmung vorgenommen wird, die so stark ist, dass mit dieser Halsabstützung eine notwendige Abziehkraft erzeugt werden kann, wobei Klemmkraft und Abziehkraft durch zwei gegenläufige Hebelarme erzeugt werden, deren Biegemomente sich im Hinblick auf die Klemmstelle kompensieren, wenn entgegengesetzt gleich grosse Kräfte an den Hebeln angreifen. Die Vorrichtung besteht aus wenigen Teilen und ist einfach zu sterilisieren. Versuche haben gezeigt, dass an den Prothesenhälsen mit der Erzeugung der Klemmkraft Marken entstehen, welche die hohe Flächenpressung wiederspiegeln. Dabei ist das Profil an den Drehkörpern so ausgelegt, dass das Produkt von Klemmkraft und Reibung immer etwas grösser ist als die notwendige Abziehkraft. Bei sehr grossen Kugeln kann die Oberkante der Klemmkörper mit der Drehung der Drehkörper direkt am Sockel der Kugel angreifen. In den meisten Fällen wird jedoch ein Zwischenkörper bevorzugt der die Abziehkräfte von der Oberkante der Drehkörper auf die Kugel überträgt.

Es ist jedoch auch möglich entsprechend dem unabhängigen Anspruch 2, die Abrollbewegung des Profils der Drehkörper direkt auf das Gehäuse in Form eines Hubes zu übertragen und mit einem entsprechend geschlitzten Gehäuse die Kugel abzuziehen.

Weitere Verbesserungen der Erfindung ergeben sich aus den abhängigen Ansprüchen 3 bis 14.

Wenn sich der Spalt S zwischen den Drehkörpern, der für die Aufnahme des Prothesenhalses vorgesehen ist, V-förmig verjüngt, können Prothesenhälse mit unterschiedlich grossen Durchmessern zum Abziehen der Kugeln eingefahren werden. Dies kann durch eine leichte Konizität der Drehkörper bei parallelen Drehachsen erreicht werden oder durch eine V-förmige Anordnung der Drehachsen und im Bereich des Spaltes S zylindrische Drehkörper. Die letztere Anordnung hat den Vorteil, dass unabhängig vom Durchmesser des Prothesenhalses gleiche drehwinkelabhängige Verhältnisse für das Klemmen und für das Anheben der Oberkante entstehen. Es genügt daher, mit einem gabelförmigen Zwischenkörper auf den Hals der Prothese aufzufahren, mit der Abziehvorrichtung darunter einzufahren, beide Instrumente leicht zur Kugel hin anzupressen und die Hebel zu betätigen, um Klemmung und Abziehen simultan zu bewirken. Bei kurzen Prothesenhälsen ist ein separater gabelförmiger Zwischenkörper von Vorteil, da dessen Schenkel sehr niedrig ausgeführt werden können, wenn sie quer zur Oberkante der Drehkörper liegen.

Man kann aber auch einen gabelförmigen Zwischenkörper beweglich am Gehäuse verankern, dessen Einfahrschlitz parallel zum Spalt zwischen den Drehkörpern verläuft. Diese Anordnung hat den Vorteil, dass keine dritte Hand zum Halten des Zwischenkörpers notwendig ist und dass der Operateur ohne zusätzliche Hilfe die Kugel abziehen kann.

Wenn die Drehkörper spiegelbildlich angeordnet sind und spiegelbildlich kinematisch miteinander verbunden sind, heben sich die Oberkanten der Drehkörper gleichzeitig und es entsteht eine symmetrische Belastung.

Die drehwinkelabhängige theoretische Spaltverringerung eines Drehkörpers ist kleiner als die Hubvergrösserung seiner Oberkante zur Ebene F. Sie bilden ein Verhältnis zwischen 0,05 und 0,5.

Der theoretischen durch die Profile vorgegebenen Verringerung der Spaltweite S wird nur begrenzt Folge geleistet, indem eine geringe plastische Deformation am Prothesenhals erzeugt wird, während praktisch ein Teil der Bewegung dazu dient, die Drehkörper und ihre Abstützung so weit elastisch auseinander zu treiben, dass eine notwendige Klemmkraft erzeugt wird.

Aus diesem Grund ist es vorteilhaft die Drehkörper mit Stützkörpern, welche als Biegeträger aus dem Gehäuse vorstehen, auf ihrer Aussenseite abzustützen.

Der Hub der Oberkante der Drehkörper sollte mehr als 0,5 mm betragen. Ein Hub von 3 mm langt für die üblichen konischen Verbindungen.

Mit der Abziehbewegung verringern die Hebelarme ihren Abstand, sodass in der Endphase beide Hebelarme mit einer Hand umspannt werden können, um die maximal notwendige Abziehkraft zu erzeugen.

Im folgenden wird die Erfindung anhand von Ausführungsbeispielen beschrieben. Es zeigen:
- Fig. 1: Schematisch eine Ansicht einer Kugelabziehvorrichtung ohne Zwischenkörper;
- Fig. 2: schematisch einen vergrösserten Schnitt durch eine Kugelabziehvorrichtung nach Figur 1 mit einem separaten Zwischenkörper nach dem Aufsetzen der Vorrichtung auf einen Prothesenhals;
- Fig. 3: schematisch die Anordnung von Figur 2 beim Lösen der konischen Verbindung zwischen Kugel und Prothesenhals;
- Fig. 4: schematisch einen vergrösserten Schnitt durch Drehkörper gemäss Figur 1, aus welchem die Verzahnung der Drehkörper und die Verankerung der Hebel in den Drehkörpern ersichtlich sind;
- Fig. 5: schematisch einen vergrösserten Schnitt durch Drehkörper gemäss Figur 1, aus dem die geometrischen Verhältnisse im Klemmbereich ersichtlich sind;
- Fig. 6: schematisch einen vergrösserten Schnitt durch eine Anordnung analog zu Figur 3, bei der ein Zwischenkörper in Abziehrichtung beweglich am Gehäuse gehalten ist;
- Fig. 7: schematisch eine weitere Ausführung, bei der das Gehäuse mit Stützarmen direkt an der aufgesetzten Kugel eine Abziehkraft erzeugt;
- Fig. 8: schematisch einen Schnitt durch die Anordnung in Figur 7; und
- Fig. 9: schematisch eine Ansicht der Drehkörper aus Figur 7 mit ihrer Verzahnung.

Die Erfindung handelt von einer Kugelabziehvorrichtung für Schaftprothesen mit auf den Prothesenhals 2 aufsetzbaren Kugeln 3. Die Vorrichtung besitzt zwei in einem Gehäuse 4 drehbar abgestützte Drehkörper 5a, 5b, die jeweils mit einem Hebelarm 6a, 6b drehbar sind und die mit ihren Drehachsen 7a, 7b in einer gemeinsamen Ebene F liegen und in der Projektion auf die Ebene F einen geöffneten Spalt S für das Einführen des Prothesenhalses 2 bilden. Die Drehkörper besitzen ein Profil, welches mit zunehmender Drehung des Spalt S zum Klemmen des Prothesenhalses 2 verringert und eine Vergrösserung des Abstandes ihrer Oberkante 9a, 9b zur Ebene F bewirkt, um direkt oder indirekt über einen Zwischenkörper an der aufgesetzten Kugel eine Abziehkraft P zu erzeugen.

Für gleiche Elemente sind in den Figuren gleiche Kennzeichen verwendet.

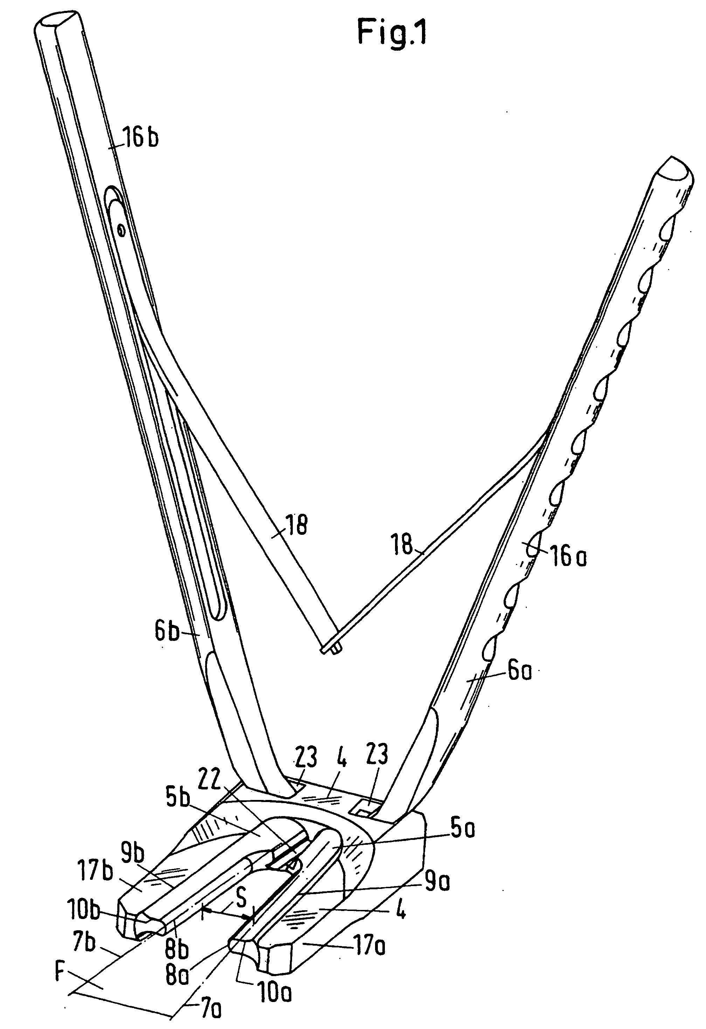

In den Figuren 1, 4 und 5 ist ein Ausführungsbeispiel gezeigt, bei dem zwei Drehkörper 5a, 5b in einem Gehäuse 4 drehbar gelagert sind. Das Gehäuse besitzt zwei Fenster 23, durch die jeweils ein Hebelarm 6a, 6b in den Drehkörper 5a, 5b hineinragt und dort mit einem Sicherungsstift 21 verankert ist. Die Drehkörper 5a, 5b überdecken sich im Bereich der Hebelarme 6a, 6b und weisen dort eine Verzahnung 22 auf, die eine begrenzte, simultane und spiegelbildliche Drehung der Drehkörper erlaubt. Die Fenster 23 sind entsprechend gross in Drehrichtung ausgeführt, während sie in axialer Richtung der Drehkörper 5a, 5b die mit den Hebelarmen 6a, 6b verbundenen Drehkörpern sichern. Die Drehkörper liegen mit einer durch einen Radius R₁ bestimmten Mantelfläche 24 am Gehäuse 4 an, sodass die Gehäusebohrungen mit einem Radius R₁ die Lage der Drehachsen 7a, 7b der Drehkörper 5a, 5b festlegen. Die Drehachsen 7a, 7b liegen in einer Ebene F und sind leicht V-förmig zueinander geöffnet, um mit einem zylindrischen Profil 8a, 8b einen sich öffnenden Spalt S zu bilden, in den Prothesenhälse mit unterschiedlichem Durchmesser bis zur seitlichen Berührung an Berührungspunkten 13a, 13b einfahrbar sind. In Figur 1 ist der Spalt S in seiner grössten Weite gezeichnet, bei der die Hebelarme 6a, 6b am weitesten auseinander stehen und durch Rückstellfedern 18 in dieser für das Einfahren der Prothesenhälse vorgesehenen Einfahrstellung gehalten sind. Die Drehkörper 5a, 5b sind auf der ganzen Länge des Spaltes S an ihrer Aussenseite an ihrer Mantelfläche 24 durch aus dem Gehäuse 4 vorstehende Stützarme abgestützt, welche als Biegefedern die beim Klemmen der Prothesenhälse erzeugten Anpresskräfte übernehmen und über das Gehäuse kompensieren. Die Profile 8a, 8b sind so ausgelegt, dass der Spalt S mit dem Drehen der Drehkörper 5a, 5b aus der Einfahrstellung verkleinert wird und gleichzeitig ein Abrollen der Profile 8a, 8b auf dem Prothesenhals erfolgt. Die Profile 8a, 8b weisen längs der Drehkörper 5a, 5b eine Oberkante 9a, 9b auf, die sich mit der Drehung relativ zur Drehachse 7a, 7b schneller nach oben bewegt als sich die Berührungspunkte relativ zur Drehachse 7a, 7b nach unten bewegen, sodass die Hubbewegung der Oberkante 9a, 9b zur Kugel hin verstärkt wird. Bei sehr grossen Kugeldurchmessern können die Oberkanten 8a, 8b direkt an der Kugel angreifen, um eine Abziehkraft zu erzeugen. Für mittlere und kleinere Kugeldurchmesser ist es zweckmässig zwischen Kugel und Oberkanten 9a, 9b einen gegabelten Zwischenkörper 11 anzuordnen, der die Kräfte auf die Kugel überträgt.

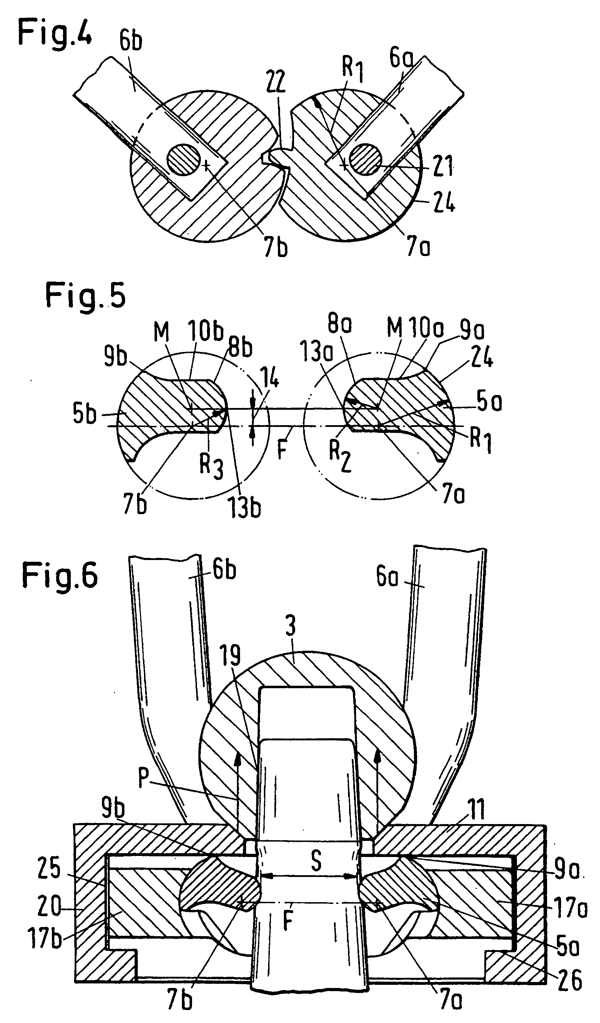

In Figur 5 ist ein Beispiel für eine Profil 8a, 8b gezeigt. Die Drehkörper 5a, 5b mit Drehachsen 7a, 7b besitzen auf ihrer Aussenseite ein Stück Mantelfläche 24 mit Radius R₁, welches bis zur Oberkante 9a, 9b ansteigt. Das eigentliche Abrollprofil 8a, 8b wird durch einen Kreisabschnitt mit Radius R₂ bestimmt dessen Mittelpunkt M um einen Betrag 14 oberhalb der Ebene F angeordnet ist. Entsprechend sind die Berührungspunkte 13a, 13b zum Prothesenhals oberhalb der Ebene F angeordnet. Diese Anordnung hat den Vorteil, dass sich der Radius R₃ eines Berührungspunktes 13a, 13b zur Drehachse 7a, 7b und der Radius R₂ des Profils in einem wählbaren Winkel schneiden, der die Zunahme der Klemmung mit der Drehung der Drehkörper 5a, 5b bestimmt.

Grundsätzlich ist es auch möglich, den Mittelpunkt M in die Ebene F zu verlegen und den Krümmungsradius R₂ grösser als R₃ zu wählen, um eine Klemmung mit zunehmender Drehung zu erreichen. Der Übergang vom eigentlichen Abrollprofil 8a, 8b zur Oberkante 9a, 9b geschieht durch eine Einbuchtung 10a, 10b.

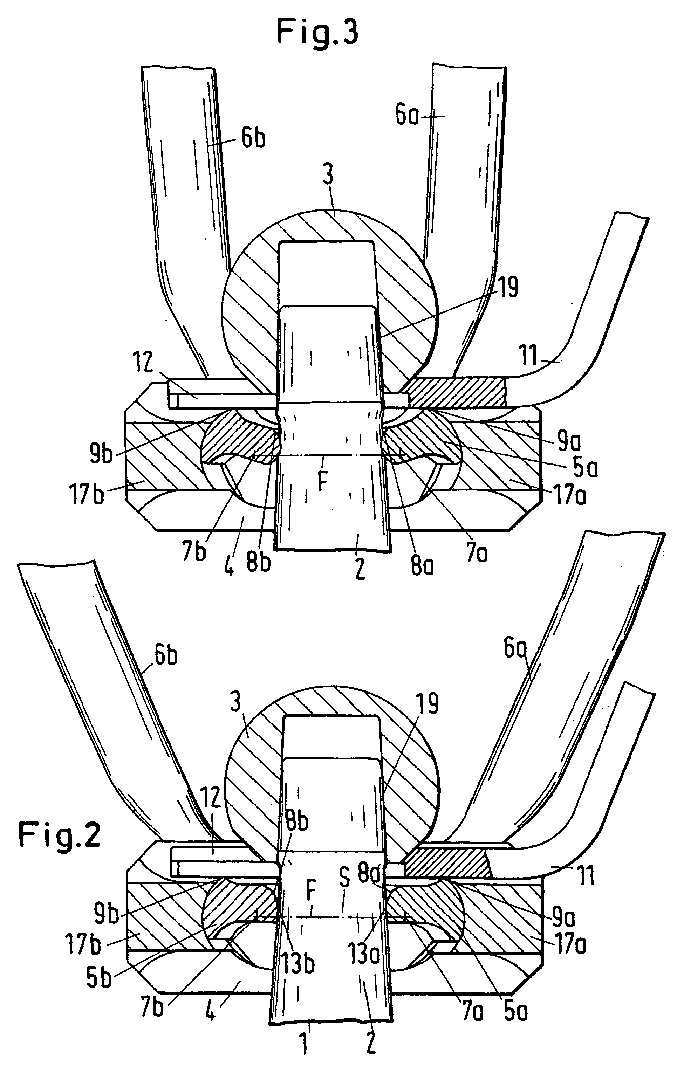

In den Figuren 2 und 3 ist das Zusammenwirken einer erfindungsgemässen Kugelabziehvorrichtung mit separatem Zwischenkörper 11 an einem Prothesenhals 2 gezeigt, der zu einer Schaftprothese 1 oder allgemein zu einem Prothesenschaft in einem Röhrenknochen gehört. Gemäss Figur 2 wird zunächst ein Zwischenkörper 11 in Form eines gabelförmigen Instruments 12 um den Prothesenhals 2 eingefahren. Anschliessend wird die Abziehvorrichtung in ihrer Einfahrstellung (Hebel 6a, 6b auseinander) quer dazu eingefahren und zur Kugel 3 hin gezogen bis einerseits die Schenkel des gabelförmigen Instruments 12 an der Kugel 3 und an den Oberkanten 9a, 9b der Drehkörper 5a, 5b anliegen und andererseits der Prothesenhals 2 an Berührungspunkten 13a, 13b im V-förmigen Spalt S anliegt. Anschliessend werden die Hebelarme 6a, 6b aufeinander zubewegt und es entsteht mit der Abrollbewegung des Profils 8a, 8b eine Klemmung des Prothesenhalses und eine Aufwärtsbewegung von den Oberkanten 9a, 9b und dem gabelförmigen Instrument 12, um die Kugel 3, die auf einem Konus 19 aufgeschoben und mit einem Schrumpfsitz verankert ist, längs ihres Aufschubweges zurückzustossen, bis sie gelöst ist. In Figur 3 ist die Kugel 3 vom Konus 19 gelöst und kann nach oben entfernt werden. Das Abrollprofil 8a, 8b hat noch etwas Reserve für eine mögliches Weiterdrehen. Die in Griffen 16a, 16b (Figur 1) fortgesetzten Hebel 6a, 6b liegen so nah beieinander, dass sie in der Endphase der Drehung mit einer Hand betätigbar sind. Der Krümmungsradius R₂ des Profils 8a, 8b ist so gewählt, dass am Prothesenhals 2 keine spürbare Schädigung während dem Klemmen und dem Abziehen der Kugel entsteht.

Ein weiteres Ausführungsbeispiel zeigt Figur 6. Klemmung und Funktion der Einzelteile sind wie in Figur 1 beschrieben. Im Gegensatz zur Figur 1 ist am Gehäuse ein Zwischenkörper 11 beweglich befestigt, der einen geöffneten Schlitz aufweist, welcher parallel zum Spalt S verläuft und etwas grösser als der Spalt S ist. Das Zwischenstück besitzt Seitenschenkel 20, die ihm eine Führung in Abzugsrichtung geben, und Endanschläge 26, die seine Aufwärtsbewegung begrenzen. Der Zwischenkörper ist schalenförmig um das Gehäuse 4 herumgeführt, um die durch die Abzugskräfte P in ihrem Abstand zu den Oberkanten 9a, 9b am Zwischenkörper 11 erzeugten Momente aufzufangen. Ein Spiel 25 der Seitenschenkel 20 zu den Stützarmen 17a, 17b ist so gross bemessen, dass die Seitenschenkel trotz der elastischen Deformation der Stützarme nicht klemmen. In der gezeichneten Darstellung ist bereits ein Grossteil der Abziehbewegung erfolgt. Ein Vorteil dieser Anordnung ist, dass der Operateur ohne fremde Hilfe die Abziehvorrichtung mit dem Zwischenkörper 11 in einer Richtung einfahren kann und die Kugel abziehen kann, ohne den Prothesenschaft im Knochen zu lockern.

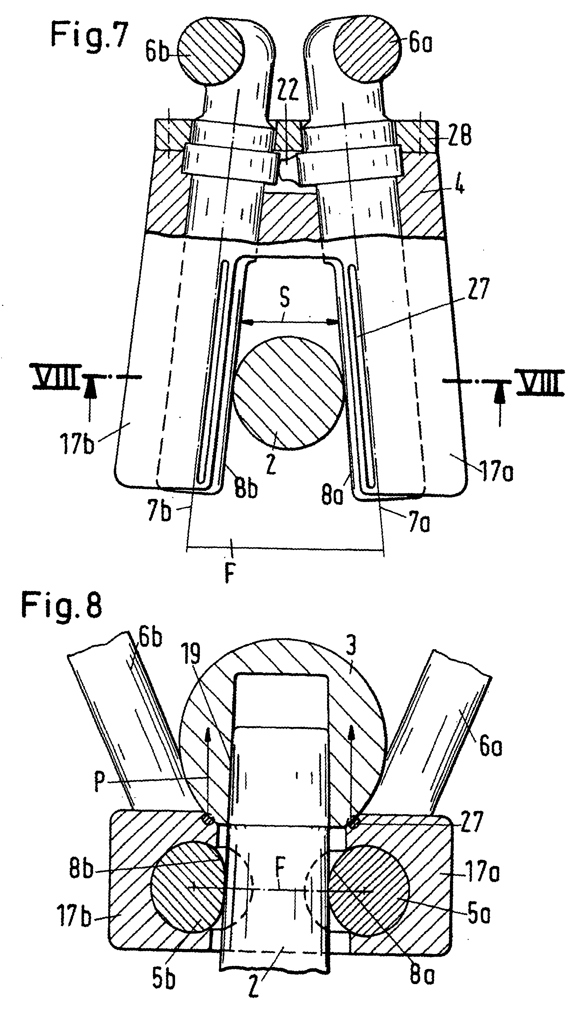

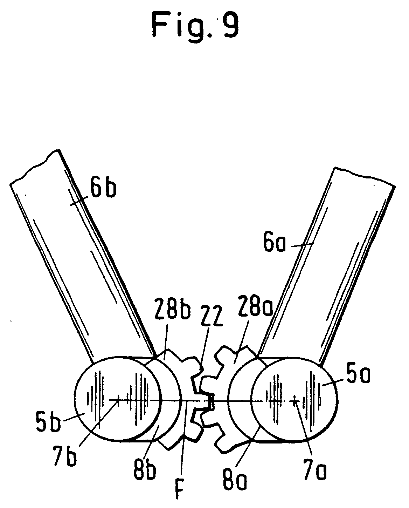

Ein weiteres Ausführungsbeispiel, das dem unabhängigen Patentanspruch 2 entspricht, ist in den Figuren 7, 8 und 9 gezeigt. Zur Erzeugung der Abziehkraft P wird nur noch die Abrollbewegung der Drehkörper 5a, 5b verwendet, welche auf das Gehäuse 4 und die Stützarme 17a, 17b als Hub übertragen wird. Aus diesem Grund sind die Drehkörper 5a, 5b auf einem grösseren Umfangswinkel vor allem auf der Oberseite umschlossen. Die Drehkörper 5a, 5b weisen ebenfalls ein Profil 8a, 8b auf, welches mit zunehmender Drehung den Spalt S verkleinert, um einen eingeschobenen Prothesenhals zu klemmen und mit den Stützarmen 17a, 17b die Kugel 3 abzuziehen. Die Drehkörper 5a, 5b sind in V-förmig angeordneten Bohrungen des Gehäuses 4 eingeschoben und werden mit einer Brille 28 in ihrer Längsrichtung gesichert. Beide Drehkörper 5a, 5b weisen jeweils einen Kragen 28a, 28b mit einer Zahnung 22 auf, welche ineinandergreifen, um spiegelbildliche Bewegungen zu erzeugen. An den Enden der Drehkörper sind Hebel 6a, 6b angeformt, um die Drehung von Hand auszuführen. Die Federwirkung der Stützarme 17a, 17b wird ebenfalls zur Erzeugung einer definierten Klemmkraft benutzt. Um ein "Durchrutschen" der Klemmkörper 5a, 5b vor dem Erreichen einer notwendigen Klemmkraft zu verringern, sind in die Stützarme 17a, 17b Federelemente 27, wie beispielsweise vorstehende Gummileisten eingelegt, die über einen Teilhub zusammenfedern bevor die eigentliche Abziehkraft P notwendig wird. Auf diese Weise kann zunächst eine genügend grosse Klemmkraft entgegen der schlagartig einsetzenden Abziehkraft aufgebaut werden.

## Patentansprüche

1. Kugelabziehvorrichtung für Schaftprothesen mit auf einen Prothesenhals (2) aufsetzbaren Kugeln (3), **gekennzeichnet durch** zwei in einem Gehäuse (4) drehbar abgestützte Drehkörper (5a, 5b), die jeweils mit einem Hebelarm (6a, 6b) drehbar sind und die mit ihren Drehachsen (7a, 7b) in einer gemeinsamen Ebene F liegen und in der Projektion auf die Ebene F einen geöffneten Spalt S für das Einführen des Prothesenhalses (2) bilden, wobei die Drehkörper (5a, 5b) ein Profil (8a, 8b) aufweisen, welches mit zunehmender Drehung den Spalt S zum Klemmen des Prothesenhalses (2) verringert und eine Vergrösserung des Abstandes ihrer Oberkante (9a, 9b) zur Ebene F bewirkt, um direkt oder indirekt über einen Zwischenkörper an der aufgesetzten Kugel (3) eine Abziehkraft P zu erzeugen.

2. Kugelabziehvorrichtung für Schaftprothesen mit auf einen Prothesenhals (2) aufsetzbaren Kugeln (3), **gekennzeichnet durch** zwei in einem Gehäuse (4) drehbar abgestützte Drehkörper (5a, 5b), die jeweils mit einem Hebelarm (6a, 6b) drehbar sind und die mit ihren Drehachsen (7a, 7b) in einer gemeinsamen Ebene F liegen und in der Projektion auf die Ebene F einen geöffneten Spalt S für das Einführen des Prothesenhalses (2) bilden, wobei die Drehkörper (5a, 5b) ein Profil (8a, 8b) aufweisen, welches mit zunehmender Drehung den Spalt S zum Klemmen des Prothesenhalses (2) verringert und auf dem Prothesenhals (2) abrollt, um mit dem Gehäuse (4) über Stützarme (17a, 17b) an der aufgesetzten Kugel (3) eine Abziehkraft P zu erzeugen.

3. Vorrichtung nach einem der Ansprüche 1 oder 2, **dadurch gekennzeichnet, dass** der Spalt S V-förmig geöffnet ist, um Prothesenhälse (2) mit unterschiedlich grossen Durchmessern einfahren zu können.

4. Vorrichtung nach einem der Ansprüche 1 bis 2, **dadurch gekennzeichnet, dass** die Drehachsen (7a, 7b) der Drehkörper V-förmig auseinander stehen.

5. Vorrichtung nach einem der Ansprüche 1 oder 3 bis 4, **dadurch gekennzeichnet, dass** ein Zwischenkörper (11) als gabelförmiges, auf den Prothesenhals (2) aufschiebbares separates Instrument (12) ausgeführt ist, welches von beiden Drehkörpern (5a, 5b) auf die Kugel (3) zu bewegbar ist.

6. Vorrichtung nach einem der Ansprüche 1 oder 3 bis 4, **dadurch gekennzeichnet, dass** ein gabelförmiger, auf den Prothesenhals (2) aufschiebbarer Zwischenkörper (11) am Gehäuse (4) beweglich verankert ist, welcher von beiden Drehkörpern auf die Kugel (3) zu bewegbar ist.

7. Vorrichtung nach einem der Ansprüche 1 bis 6, **dadurch gekennzeichnet, dass** die Drehkörper (5a, 5b) kinematisch zum Beispiel durch eine Verzahnung (22), miteinander verbunden sind, um spiegelbildliche Bewegungen zueinander auszuführen.

8. Vorrichtung nach einem der Ansprüche 1 oder 3 bis 7, **dadurch gekennzeichnet, dass** das Verhältnis der Spaltverringerung eines Drehkörpers (5a, 5b) in seiner Projektion auf die Ebene F zur Hubvergrösserung seiner Oberkante relativ zur Ebene F zwischen 0,05 und 0,5 beträgt.

9. Vorrichtung nach Anspruch 8, **dadurch gekennzeichnet, dass** der Hub der Oberkante mehr als 0,5 mm vorzugsweise 3 mm beträgt.

10. Vorrichtung nach einem der Ansprüche 8 oder 9, **dadurch gekennzeichnet, dass** in einer Einfahrstellung für den Prothesenhals (2) die Berührungspunkte (13a, 13b) der Drehkörper (5a, 5b) am Prothesenhals um einen Betrag (14) von weniger als 6 mm von der Ebene F wegstehen, um im Rahmen einer elastischen Deformation der Drehkörper (5a, 5b) mit ihrer Abstützung und einer teilweise plastischen Deformation am Prothesenhals (3) eine Abrollbewegung der Drehkörper (5a, 5b) am Prothesenhals (3) zu erzeugen.

11. Vorrichtung nach einem der Ansprüche 1 bis 10, **dadurch gekennzeichnet, dass** die Hebelarme (6a, 6b) beim Einfahren des Prothesenhalses (2) weiter auseinander stehen als bei einem anschliessenden Drehen der Drehkörper (5a, 5b).

12. Vorrichtung nach einem der Ansprüche 1 oder 3 bis 11, **dadurch gekennzeichnet, dass** die Hebelarme (6a, 6b) sich annähernd berühren, wenn ein maximal vorgesehener Hub (15a, 15b) der Oberkante (9a, 9b) der Drehkörper (5a, 5b) von der Ebene F weg erreicht ist.

13. Vorrichtung nach einem der Ansprüche 1 bis 12, **dadurch gekennzeichnet, dass** die Drehkörper (5a, 5b) durch elastisch federnde aus dem Gehäuse (4) vorstehende Stützarme (17a, 17b) abgestützt sind, die mit ihrer Federwirkung die mit der Vergrösserung des Profils (8a, 8b) verbundene Klemmkraft bestimmen.

14. Vorrichtung nach Anspruch 2, **dadurch gekennzeichnet, dass** an den Stützarmen (17a, 17b) Federelemente (27) eingelegt sind, die beim Erzeugen einer Abziehkraft P zwischen Kugel (3) und Stützarmen (17a, 17b) zunächst zusammenpressbar sind, um während dieses Teilhubes des Gehäuses (4) eine grössere Klemmkraft zu erzeugen.

## Claims

1. Ball puller apparatus for shaft prostheses with balls (3) which can be placed onto a neck (2) of a prosthesis, **characterized by** two rotary bodies (5a, 5b) which are rotatably supported in a housing (4), which can in each case be rotated by a lever arm (6a, 6b) and whose axes of rotation (7a, 7b) lie in a common plane F and form an open gap S in the projection onto the plane F for the introduction of the neck (2) of the prosthesis, with the rotary bodies (5a, 5b) having a profile (8a, 8b) which, with increasing rotation, decreases the gap S for clamping the neck (2) of the prosthesis and causes an increase of the spacing of its upper edge (9a, 9b) from the plane F in order to produce a pull-off force P at the mounted ball (3) directly or indirectly via an intermediate body.

2. Ball puller apparatus for shaft prostheses with balls (3) which can be placed onto a neck (2) of a prosthesis, **characterized by** two rotary bodies (5a, 5b) which are rotatably supported in a housing (4), which can in each case be rotated by a lever arm (6a, 6b) and whose axes of rotation (7a, 7b) lie in a common plane and form an open gap S in the projection onto the plane F for the introduction of the neck (2) of the prosthesis, with the rotary bodies (5a, 5b) having a profile (8a, 8b) which, with increasing rotation, decreases the gap S for clamping the neck (2) of the prosthesis and rolls off on the neck (2) of the prosthesis in order to produce a pull-off force P at the mounted ball (3) with the housing (4) via support arms (17a, 17b).

3. Apparatus in accordance with any one of the claims 1 or 2, **characterized in that** the gap S is open in V shape in order to be able to move in necks (2) of a prosthesis with diameters of different sizes.

4. Apparatus in accordance with any one of the claims 1 to 2, **characterized in that** the axes of rotation (7a, 7b) of the rotary body stand apart in V shape.

5. Apparatus in accordance with any one of the claims 1 or 3 to 4, **characterized in that** an intermediate body (11) is designed as a separate fork-like instrument (12) which can be pushed onto the neck (2) of the prosthesis and which can be moved toward the ball (3) by both rotary bodies (5a, 5b).

6. Apparatus in accordance with any one of the claims 1 or 3 to 4, **characterized in that** a fork-like intermediate body (11), which can be pushed onto the neck (2) of the prosthesis and which can be moved toward the ball (3) by both rotary bodies (5a, 5b,) is movably anchored at the housing (4).

7. Apparatus in accordance with any one of the claims 1 to 6, **characterized in that** the rotary bodies (5a, 5b) are kinematically connected to one another, for example, by a toothed arrangement (22) in order to carry out mirror-image movements with respect to one another.

8. Apparatus in accordance with any one of the claims 1 or 3 to 7, **characterized in that** the ratio of the gap reduction of a rotary body (5a, 5b) in its projection onto the plane F to the stroke increase of its upper edge relative to the plane F amounts to between 0.05 and 0.5.

9. Apparatus in accordance with claim 8, **characterized in that** the stroke of the upper edge amounts to more than 0.5 mm, preferably 3 mm.

10. Apparatus in accordance with claim 8 or claim 9, **characterized in that**, in a moved-in position for the neck (2) of the prosthesis, the points of contact (13a, 13b) of the rotary bodies (5a, 5b) at the neck of the prosthesis stand off from the plane F at the neck of the prosthesis by an amount (14) of less than 6 mm in order to produce a roll-off movement of the rotary bodies (5a, 5b) at the neck (3) of the prosthesis in the context of an elastic deformation of the rotary bodies (5a, 5b) with their support and of a partly plastic deformation at the neck (3) of the prosthesis.

11. Apparatus in accordance with any one of the claims 1 to 10, **characterized in that** the lever arms (6a, 6b) stand further apart during the moving in of the neck (2) of the prosthesis than in a subsequent rotation of the rotary bodies (5a, 5b).

12. Apparatus in accordance with any one of the claims 1 or 3 to 11, **characterized in that** the lever arms (6a, 6b) almost contact one another when a maximally provided stroke (15a, 15b) of the upper edge (9a, 9b) of the rotary bodies (5a, 5b) away from the plane F has been reached.

13. Apparatus in accordance with any one of the claims 1 to 12, **characterized in that** the rotary bodies (5a, 5b) are supported by elastically resilient support arms (17a, 17b) which project out of the housing (4) and which determine with their spring action the clamping force which is associated with the increase of the profile (8a, 8b).

14. Apparatus in accordance with claim 2, **characterized in that** spring elements (27) are inserted at the support arms (17a, 17b) which can initially be pressed together during the production of a pull-off force P between the ball (3) and the support arms (17a, 17b) in order to produce a greater clamping force during this partial stroke of the housing (4).

## Revendications

1. Dispositif de retrait de rotules pour prothèses à broche, comportant des rotules (3) pouvant être emboîtées sur un col de prothèse (2), **caractérisé par** deux corps rotatifs (5a, 5b) supportés en rotation dans un boîtier (4), qui peuvent être tournés respectivement avec un bras de levier (6a, 6b), qui sont situés par leurs axes de rotation (7a, 7b) dans un plan commun F, et qui forment en projection sur le plan F une fente ouverte S pour l'insertion du col de prothèse (2), les corps rotatifs (5a, 5b) ayant un profil (8a, 8b) qui, au fur et à mesure de l'augmentation de la rotation, réduit la fente S pour le serrage du col de prothèse (2) et provoque un agrandissement de l'écartement de leur bord supérieur (9a, 9b) par rapport au plan F afin d'engendrer directement ou indirectement, par l'intermédiaire d'un corps intermédiaire, une force de retrait P sur la rotule (3) emboîtée.

2. Dispositif de retrait de rotules pour prothèses à broche, comportant des rotules (3) pouvant être emboîtées sur un col de prothèse (2), **caractérisé par** deux corps rotatifs (5a, 5b) supportés en rotation dans un boîtier (4), qui peuvent être tournés respectivement avec un bras de levier (6a, 6b), qui sont situés par leurs axes de rotation (7a, 7b) dans un plan commun F, et qui forment en projection sur le plan F une fente ouverte S pour l'insertion du col de prothèse (2), les corps rotatifs (5a, 5b) ayant un profil (8a, 8b) qui, au fur et à mesure de l'augmentation de la rotation, réduit la fente S pour le serrage du col de prothèse (2) et se déroule sur le col de prothèse (2) afin d'engendrer avec le boîtier (4), par l'intermédiaire de bras de support (17a, 17b), une force de retrait P sur la rotule (3) emboîtée.

3. Dispositif selon l'une des revendications 1 ou 2, **caractérisé en ce que** la fente S est ouverte en forme de V afin de pouvoir insérer des cols de prothèse (2) de différents diamètres.

4. Dispositif selon l'une des revendications 1 ou 2, **caractérisé en ce que** les axes de rotation (7a, 7b) des corps rotatifs sont écartés en forme de V.

5. Dispositif selon l'une des revendications 1 ou 3 à 4, **caractérisé en ce qu'**un corps intermédiaire (11) est agencé en tant qu'instrument (12) séparé, en forme de fourche, coulissant sur le col de prothèse (2), lequel peut être déplacé vers la rotule (3) par les deux corps rotatifs (5a, 5b).

6. Dispositif selon l'une des revendications 1 ou 3 à 4, **caractérisé en ce qu'**un corps intermédiaire (11) en forme de fourche coulissant sur le col de prothèse (2) est ancré de façon mobile sur le boîtier (4), et peut être déplacé vers la rotule (3) par les deux corps rotatifs.

7. Dispositif selon l'une des revendications 1 à 6, **caractérisé en ce que** les corps rotatifs (5a, 5b) sont reliés entre eux par voie cinématique, par exemple par une denture (22), afin d'exécuter des mouvements symétriques l'un par rapport à l'autre.

8. Dispositif selon l'une des revendications 1 ou 3 à 7, **caractérisé en ce que** le rapport entre la réduction de fente d'un corps rotatif (5a, 5b) dans sa projection sur le plan F et l'agrandissement du relèvement de son bord supérieur par rapport au plan F est compris entre 0,05 et 0,5.

9. Dispositif selon la revendication 8, **caractérisé en ce que** le relèvement du bord supérieur est de plus de 0,5 mm, de préférence de 3 mm.

10. Dispositif selon l'une des revendications 8 ou 9, **caractérisé en ce que**, dans une position d'insertion pour le col de prothèse (2), les points de contact (13a, 13b) des corps rotatifs (5a, 5b) sur le col de prothèse (3) sont écartés du plan F d'une valeur (14) inférieure à 6 mm, afin d'engendrer par leur support dans le cadre d'une déformation élastique des corps rotatifs (5a, 5b) et d'une déformation plastique partielle au niveau du col de prothèse (2), un mouvement de déroulement des corps rotatifs (5a, 5b) sur le col de prothèse (2).

11. Dispositif selon l'une des revendications 1 à 10, **caractérisé en ce que**, lors de l'insertion du col de prothèse (2), les bras de levier (6a, 6b) sont plus écartés l'un de l'autre que lors d'une rotation consécutive des corps rotatifs (5a, 5b).

12. Dispositif selon l'une des revendications 1 ou 3 à 11, **caractérisé en ce que** les bras de levier (6a, 6b) se touchent approximativement lorsqu'un relèvement maximal prévu (15a, 15b) du bord supérieur (9a, 9b) des corps rotatifs (5a, 5b) est atteint par rapport au plan F.

13. Dispositif selon l'une quelconque des revendications 1 à 12, **caractérisé en ce que** les corps rotatifs (5a, 5b) sont supportés par des bras de support (17a, 17b) élastiquement flexibles dépassant du boîtier (4) qui définissent par leur effet d'élasticité la force de serrage associée à l'agrandissement du profil (8a, 8b).

14. Dispositif selon la revendication 2, **caractérisé en ce que** des éléments élastiques (27) sont insérés dans les bras de support (17a, 17b) qui, lors de la génération d'une force de retrait P, peuvent dans un premier temps être comprimés entre la rotule (3) et les bras de support (17a, 17b), afin de générer une force de serrage plus importante lors de ce relèvement partiel du boîtier (4).
